Europäisches Patentamt

European Patent Office    (11) Publication number: **0 092 528**

Office européen des brevets    **A1**

(12) **EUROPEAN PATENT APPLICATION**

(21) Application number: **83830068.9**    (51) Int. Cl.³: **A 61 M 1/03**

(22) Date of filing: **29.03.83**

(30) Priority: **16.04.82 IT 2079282**

(71) Applicant: **SIS-TER S.p.A., Via Crema, 14, I-26020 Palazzo Pignano (Cremona) (IT)**

(43) Date of publication of application: **26.10.83 Bulletin 83/43**

(72) Inventor: **Bertellini, Gianfranco, Via XX Settembre, 58, Maslianico (Como) (IT)**
Inventor: **Ghezzi, Paolo, Frazione S. Lorenzo, 151 Peverogno (Cuneo) (IT)**

(74) Representative: **Vannini, Torquato et al, JACOBACCI-CASETTA & PERANI S.p.A. 7 Via Visconti di Modrone, I-20122 Milan (IT)**

(84) Designated Contracting States: **AT BE CH DE FR GB LI LU NL SE**

(54) Endoperitoneal dialysis device.

(57) An endoperitoneal dialysis device is provided which avoids the danger of infection resulting from handling by the subject and also avoids the possibility of disinfectant being introduced into the peritoneum. The device includes a sack (2) and a connecting tube (3) for connecting the sack (2) to the catheter (4). The sack (2) has main and auxiliary chambers (6, 9) respectively provided with tubular mouth parts (15, 16). The mouth part (15) of the main chamber (8) has a rupturable septum (18) and a branch (19) which in its turn is provided with a pierceable plug (20) located downstream of the rupturable septum (18). The external ends of the said tubular mouth parts (13, 16) are respectively provided with male and female connectors (21, 23) by means of which the mouth parts (15, 16) can be connected to each other. The said connecting tube (3) branches in the form of a Y into two tube sections (30, 31) having respective end connectors (32, 33) which are releasably connectable not only to each other but also to the connectors (21, 23) of the tubular mouth parts (15, 16). Closure means (33, 25) are provided on the connecting tube (3) and on the tubular mouth part (16) of the auxiliary chamber (9).

- 1 -

## Endoperitoneal Dialysis Device.

The present invention relates to the field of endoperitoneal dialysis and particularly to an endoperitoneal dialysis device comprising a sack and a tube for connecting the sack to a catheter.

As is known, for people whose natural purifying function is inactive or insufficient, it is necessary to carry out dialysis by feeding a suitable saline dialysis solution into the peritoneum, this process being, to advantage, carried out by the subject himself without medical supervision.

In order to satisfy this requirement, at present the subject is provided with a catheter inserted in the peritoneum and he uses a sack containing a saline dialysis solution which he connects to the catheter through a connecting tube of suitable length.

The sack contents are made to flow into the peritoneum where they fulfill the required dialysis.

On average the subject uses four sacks a day and replaces the connecting tube with a new one about once a month.

The device specified above, although advantageous from various points of view, suffers from a serious disadvantage in the danger of infection resulting from daily handling by the subject in order to connect the sack to and disconnect it from the connecting tube and the monthly handling or replacing of the connecting tube by new ones.

In order to avoid this disadvantage, sacks have been proposed which are provided with a small compartment containing a liquid disinfectant, for example polyvinyl pyrrolidone iodide or chlorhexidine or hypochlorite, which ensures that the tube is disinfected.

However such devices have the disadvantage of causing disinfectant to be fed into the peritoneum with all the contra-indications consequent thereon.

The problem which is at the basis of the present invention is that of devising a device of the type specified which has structural and functional characteristics such as to satisy the said dialysis needs, which can to advantage be used by the subject himself without medical supervision, while at the same time overcoming all the disadvantages mentioned with reference to the known art.

This problem is resolved by a device of the type specified which is characterised in that the sack comprises a main chamber provided with a tubular mouth part having a rupturable septum and a branch which in its turn is provided with a pierceable plug located downstream of the rupturable septum, an auxiliary chamber provided with a tubular mouth part, and a male connector and female connector respectively provided at the end of a corresponding one of the tubular mouth parts and enabling the releasable interconnection of said mouth parts, the connecting tube branching at one end in the form of a Y into two tube sections respectively provided with male and female end connectors connectable not only to each other but also to the connectors of the tubular mouth part, closure means being provided for the connecting tube and the tubular mouth part of the said auxiliary chamber.

Further characteristics and advantages of the device according to the present invention will emerge from the following description of a preferred embodiment given by way of non-limiting example, with reference to the appended drawings, in which:

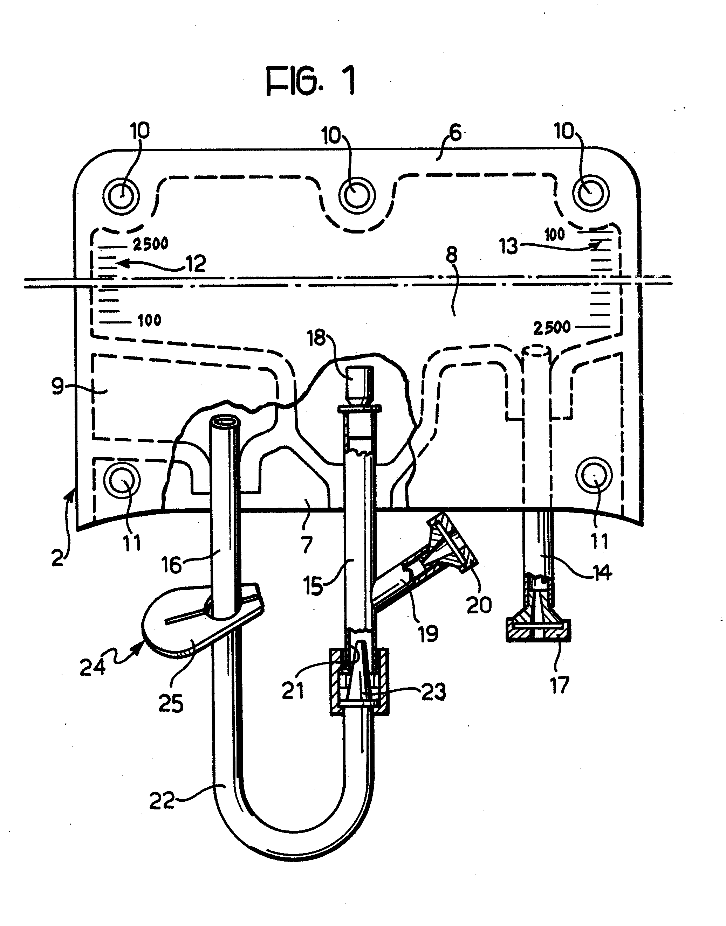

- Figure 1 is a partially sectioned, perspective view of a sack of a device according to the invention,
- Figure 2 is a perspective view of a connecting tube of a device according to the invention, and
- Figures 3 to 10 are schematic views of the device of Figures 1 and 2 in successive phases of its operation.

With reference to the appended drawings, an endoperitoneal dialysis device generally indicated 1 comprises a sack 2 and a tube 3 for connecting the sack 2 to a catheter 4 inserted in a peritoneum 5.

The sack 2 is formed from a transparent, tubular, PVC envelope having ends 6 and 7 heat sealed to define a main chamber 8 occupying almost the whole of the sack and containing the saline dialysis solution, and an auxiliary chamber 9 occupying the remaining part of the sack in a position adjacent a part of the end 7.

The end 6 of the sack 2 has holes 10 for suspending the sack itself in a position higher than the catheter 4.

The end 7 of the sack 2 has holes 11 for suspending the sack in a position lower than the catheter 4.

The sack 2 is provided along its length with two graduated scales 12, 13 respectively increasing from the end 7 to the end 6 and visa versa.

At the end 7 of the sack 2 the main chamber 8 is provided with a tubular mouth part 14 and a tubular mouth part 15 while the auxiliary chamber 9 is provided a tubular mouth part 16.

The tubular mouth parts 14, 15 and 16 are formed from cut pieces of tube retained in the sack 2 during heat sealing.

More particularly, the tubular mouth part 14 has a piercable plug 17 at its end which can be pierced by a needle of a syringe, not shown, for the optional insertion into the chamber 8 of medicinal substances such as insulin, as an addition to the saline dialysis solution.

The tubular mouth part 15 is provided internally with a rupturable septum 18 and has a branch 19 downstream of the rupturable septum 18 which in its turn is provided with a piercable plug 20. A connector indicated 21 is located at the end of the tubular mouth part 15 and is advantageously of the female LUER-LOCK type.

The tubular mouth part 16 is formed from a flexible tube 22 of predetermined length greater than the length of the tubular mouth part 15 and having a connector 23 at its end which advantageously is of the male LUER-LOCK type.

The connectors 21 and 23 are releasably interconnectable, the overall length of the flexible tube 22 being chosen so as to allow the connector 23 to be connected to the connector 21.

The tubular mouth part 16 is provided with closure means 24, advantageously constituted by a pincer 25 of the type known as an ON-OFF pincer, which surrounds the flexible tube 22.

The connecting tube 3 of the device 1 according to the invention has an end 26 connectable, by a male/female coupling, to the free end of the catheter 4, this coupling being advantageously implemented by means of LUER-LOCK connectors 27 and 28; the opposite end of the tube branches in the form of a Y into two tube sections of different length 30 and 31. The arm 30 is longer and has a connector 32, advantageously of the male LUER-LOCK type, while the shorter arm 31 has a connector 33 which is advantageously of the female LUER-LOCK type.

The connectors 32 and 33 are interconnectable by means of their male and female portions, and may also be connected to the connectors 21 and 23.

The sections 30 and 31 are of different lengths so as to allow for the different lengths of the tubular mouth parts 15 and 16.

The connecting tube 3 is provided with closure means 34, advantageously constituted by an ON-OFF pincer 35 placed between the end 26 and the branch point.

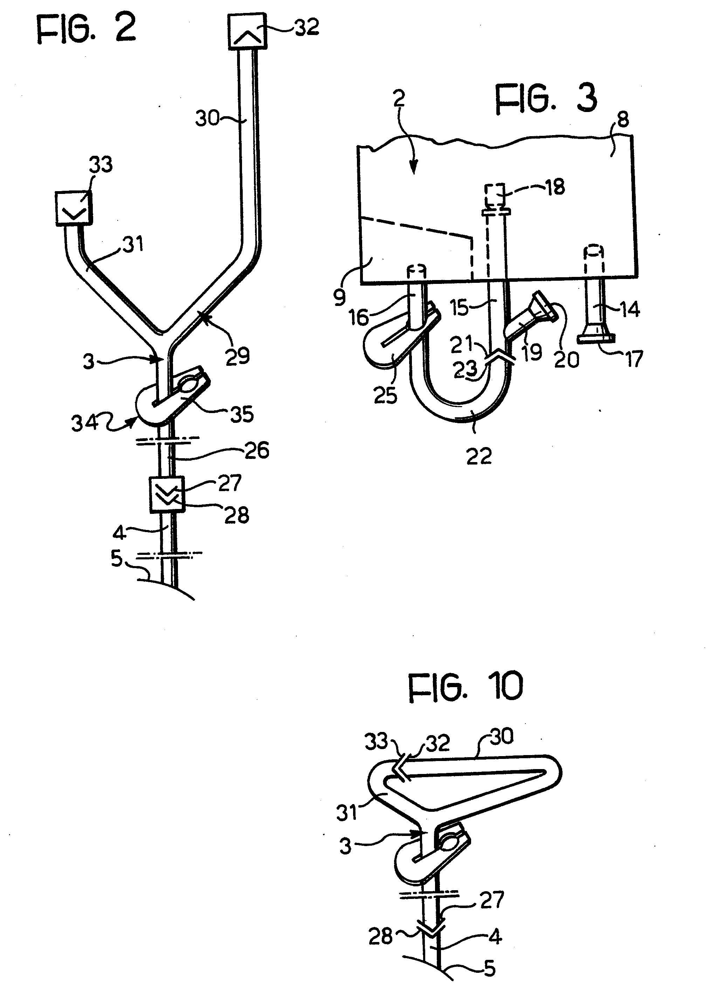

The operation of the device 1 according to the invention is described below with reference to an initial condition such as that illustrated in Figure 3, in which the sack 2 alone is shown, in customary pharmacological manner, while the connecting tube 3 is arranged as illustrated in Figure 2, connected to the catheter 4.

In particular, the tubular mouth parts 15 and 16 of the sack 2 are placed in communication by means of the male and female connectors 21 and 23. The rupturable septum 18 is unbroken so that the tubular mouth part 15 is closed. The ON-OFF pincer 25 is in its open position so that the passage through the flexible tube 22 of the tubular mouth part 16 is open. In the sack 2 the main chamber 8 is full of saline dialysis solution while the auxiliary chamber 9 contains only a small quantity of saline dialysis solution to the extent necessary to prevent it becoming sealed up during steralization to which the sack is subjected after filling. The presence of saline solution is indicated by the "+" signs in the drawings.

The ON-OFF pincer 35 of the connecting tube 3
(see Figure 2) is in its closed position so that
passage through this tube 3 is prevented.

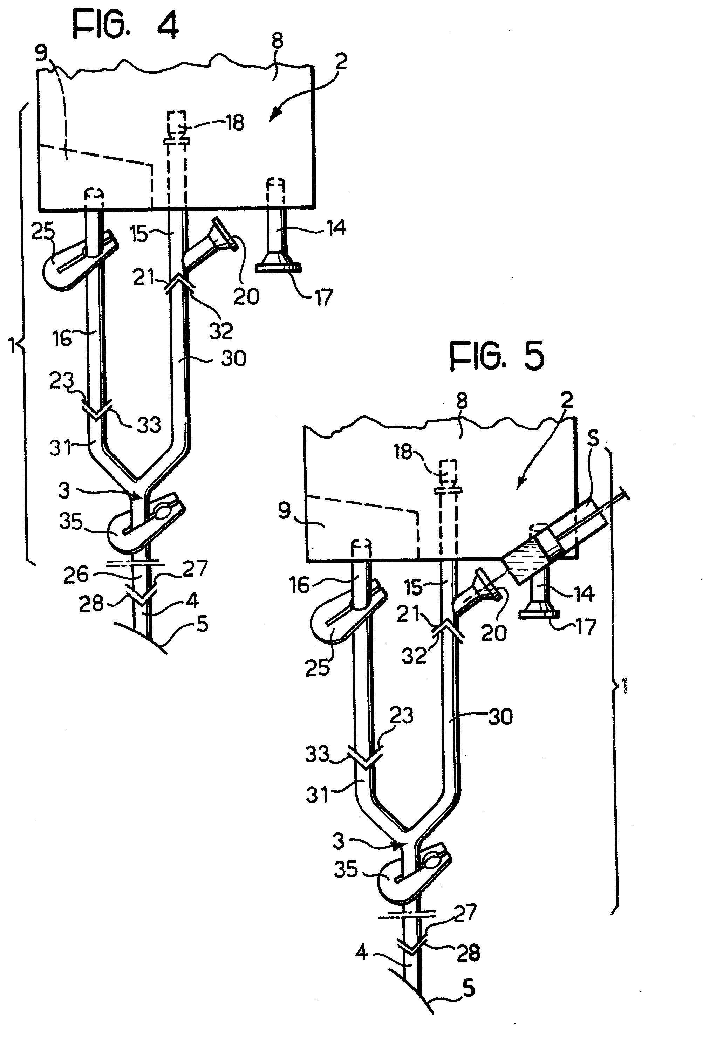

At this point (see Figure 4) the subject
disconnects the connectors 23 and 21 and connects
them to the bodies 33 and 32 respectively: this
connects the sack 2 to the catheter 4 through
the connecting tube 3. Saline solution thus flows
into the connecting tube 3 as far as the ON-OFF
pincer 35.

At this point (see Figure 5) the subject,
with the aid, for example, of a syringe S, injects
a liquid disinfectant through the piercable plug
20, the membrane of which is easily penetrated by
the needle of the syringe, and the disinfectant
fills the  auxiliary chamber 9 mixing with the
saline solution contained therein as well as with
the saline solution contained in the tubular mouth
parts 15 and 16 and in the tube 3 up to the ON-OFF
pincer 35. The presence of liquid disinfectant is
indicated by the "-" signs in the drawings. Clearly,
the desired disinfection . of the connectors 23 and
33and of the connectors 21 and 32, as well as of the
tubular mouth parts 15 and 16 and the tube sections
30 and 31 is thus effected so as to neutralise any
bacilli introduced during manipulation of the
connectors by the subject while connecting them.

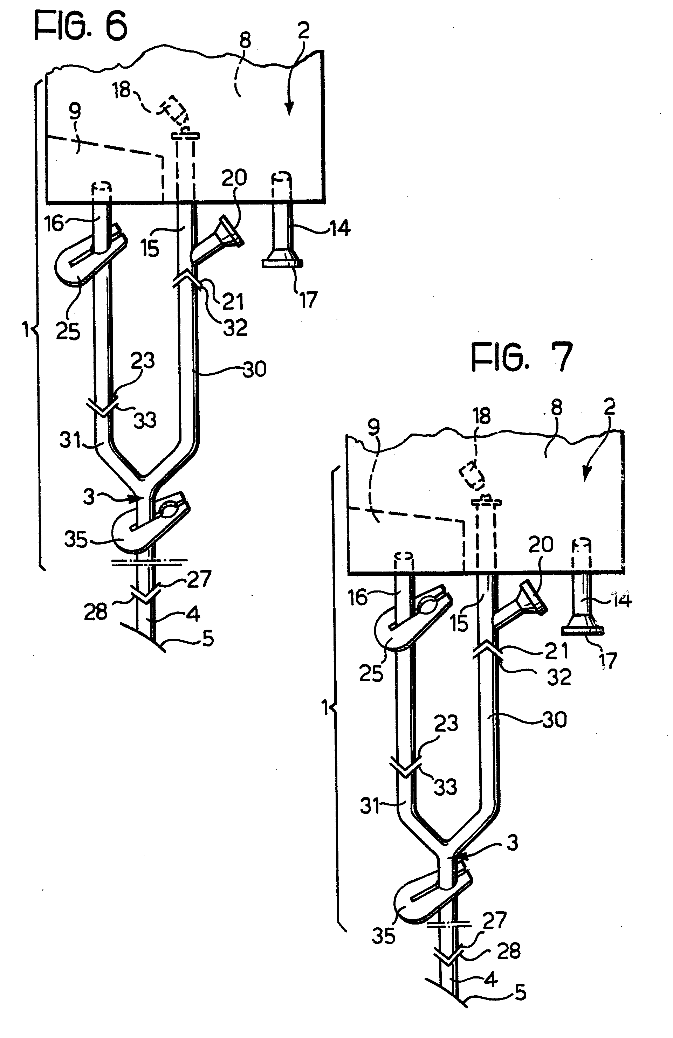

The subject then (see Figure 6) suspends the
sack  by  the holes 10, breaks the rupturable
septum 18 and causes the saline solution contained
in chamber 8 to flow towards the chamber 9 thereby
forcing the mixture of saline solution and liquid
disinfectant contained in the tubular mouth part 15,
the tubular sections 30 and 31, and the tubular
mouth part 16, into the chamber 9.

After this has been done, the subject (see Figure 7) moves the ON-OFF pincer 25 into its closed position and places the ON-OFF pincer 35 in its open position. In this manner, the mixture of saline solution and liquid disinfectant is trapped in the chamber 9 while saline solution can flow freely from the chamber 8 into the peritoneum.

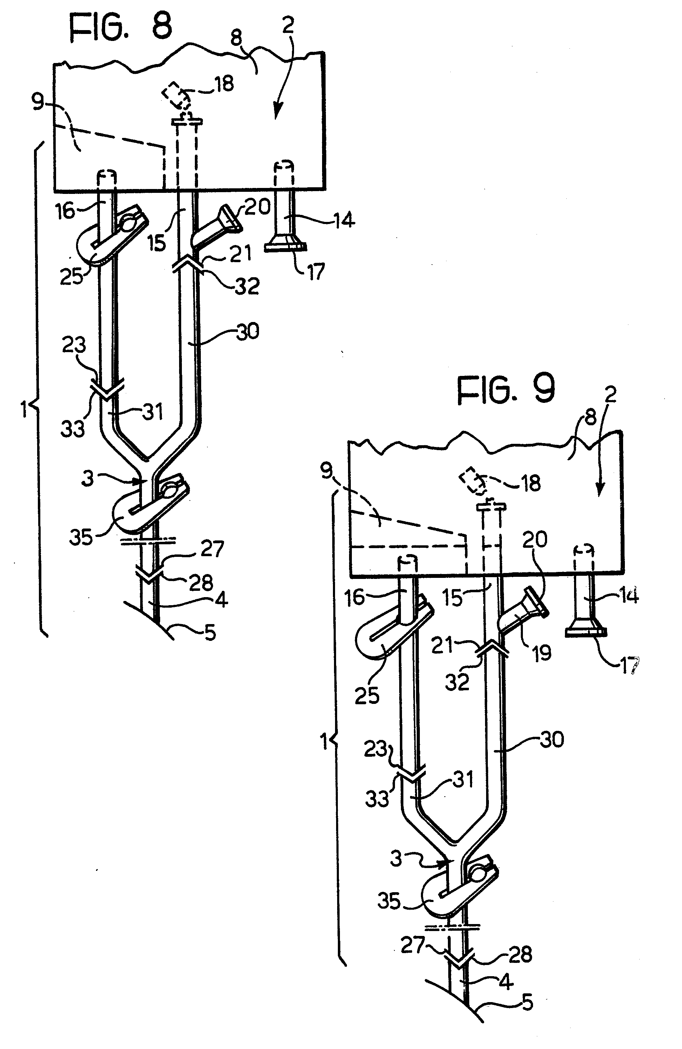

When the contents of the main chamber 8 are exhausted, the subject moves the ON-OFF pincer 35 into its closed position (see Figure 8) and moves the ON-OFF pincer 25 into its open position (see Figure 9) thereby causing the mixture of saline solution and disinfectant to flow out of the auxiliary chamber 9 towards the main chamber 8 through the tubular mouth part 16, the tube sections 30 and 31, and the tubular mouth part 15 until it reaches the same level in the two chambers 8 and 9 (see Figure 9). At this point the subject disconnects the connectors 23 and 33 and connects together the connectors 21 and 32, throws away the sack 2 and connects together the connectors 32 and 33, thereby joining together the tube sections 30,31 of the of the connecting tube 3 (see Figure 10).

Tube sections 30 and 31 of the tube 3 thus remain filled down to the ON-OFF pincer 35 with the mixture of saline solution and disinfectant. This mixture ensures the complete disenfection of the connectors 32,33 from any bacilli introduced during handling fo the connectors themselves by the subject.
The connecting tube 3 may then be retained by the subject beneath his clothing,the tube remaining disinfected due to the presence therein of the mixture until dialysis is again effected using a new sack 2 after draining off of the peritoneal fluid.

Clearly, as an alternative to what has been described above, when the level of liquid in the chambers 8 and 9 is the same (see Figure 9), the sack 2, instead of being thrown away, may be folded up and retained by the subject under his clothing to be used later, suspended by the holes 11, for draining off liquid when dialysis has been completed, it being understood that the ON-OFF pincer 35 would first be moved into its open position.

Once drainage has been carried out, the sack is disconnected and thrown away, and a new sack is connected.

From what has been described above, it is clear that the device according to the invention allow endoperitoneal dialysis to be carried out while completely eliminating the danger of infections resulting from the handling of it connectors, and also avoiding completely the introduction of liquid disinfectant into the peritoneum.

The device according to the invention also renders less stringent the measures used up to now to maintain the sterility of the outer surfaces of the parts subject to handling.

0092528

## CLAIMS

1.  An endoperitoneal dialysis device comprising
a sack (2) and a connecting tube (3) for connecting
the sack (2) to a catheter (4), characterised in
that the sack (2) comprises

   - a main chamber (8) having a tubular mouth part
provided with a rupturable septum (18) and a branch
(19) which in its turn is provided with a pierceable
plug (20) located downstream of the rupturable septum
(18),

   - an auxiliary chamber (9) provided with a
tubular mouth part (16), and

   - a male connector (23) and female connector (21)
respectively provided at the external end of a corresponding one of the
said tubular mouth parts (15, 16) and enabling the
releasably interconnection of the mouth parts,
the said connecting tube (3) branching at one end in
the form of a Y into two tube sections (30, 31)
respectively provided with male and female end
connectors (32, 33) releasably connectable not
only to each other but also to the connectors (21,23)
of the tubular mouth parts (15, 16), and closure
means (34, 24) being provided for the connecting
tube (3) and the tubular mouth part (16) of the
auxiliary chamber (9).

# FIG. 1

0092528

FIG. 2

FIG. 3

FIG. 10

FIG. 4

FIG. 5

0092528

4/5

FIG. 6

FIG. 7

0092528

5/5

FIG. 8

FIG. 9

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | EP-A-0 029 526   (S.I.F.R.A.)<br>* Whole document, in particular figures 1,14; page 9, lines 12-17; page 10, lines 2-4 * | 1 | A 61 M   1/03 |
| A | DE-A-2 809 303   (POPOVICH)<br>* Whole document, in particular figure 2, reference sign 45 * | 1 | |
| A | EP-A-0 049 525   (SIS-TER)<br>* Whole document, in particular figure 1 * | 1 | |
| A | US-A-4 209 013   (BAXTER)<br>* Whole document, in particular figure 10 and figure 17, reference sign 80 * | 1 | |
| A | FR-A-2 440 740   (ABG-SEMCA)<br>* Figure 1; page 10, lines 3-15, 23-25 * | 1 | TECHNICAL FIELDS SEARCHED (Int. Cl. ³)<br><br>A 61 M |

The present search report has been drawn up for all claims

| Place of search | Date of completion of the search | Examiner |
|---|---|---|
| THE HAGUE | 08-07-1983 | PETZUCH M. |